# EUROPÄISCHE PATENTSCHRIFT

(11) **EP 0 624 360 B1**
(45) Veröffentlichungstag und Bekanntmachung des Hinweises auf die Patenterteilung: **28.02.2001**
(21) Anmeldenummer: 94810268.6
(22) Anmeldetag: 06.05.1994
(51) Int. Cl.: A61K 6/06, A61L 27/00, C04B 35/48

(54) **Werkstoff für Prothesen**
Material for prostheses
Matériau pour prothèses

(30) Priorität: 07.05.1993 CH 141093
(43) Veröffentlichungstag der Anmeldung: 17.11.1994
(73) Patentinhaber: METOXIT AG, CH-8240 Thayngen (CH)
(72) Erfinder: Rieger, Dr. Wolfhart, CH-8263 Buch (CH)
(74) Vertreter: Peege, Klaus

(56) Entgegenhaltungen:
- EP-A- 0 218 853
- EP-A- 0 262 299
- DE-U- 8 708 806
- US-A- 4 328 296
- DATABASE WPI Section Ch, Week 8718, Derwent Publications Ltd., London, GB; Class D21, AN 87-125072 & JP-A-62 065 972 (SHINAGAWA FIRE BRICK) 25. März 1987
- R.J.BROOK 'CONCISE ENCYCLOPEDIA OR ADVANCED CERAMIC MATERIALS' 1990 , PERGAMON PRESS , OXFORD * Seite 526, linke Spalte, Absatz 7 *

## Beschreibung

Die Erfindung betrifft einen keramischen Werkstoff zur Herstellung von Prothesen nach dem Oberbegriff des Patentanspruches 1.

Zur Herstellung von Prothesen (Endoprothesen, Exoprothesen) sind metallische und keramische Werkstoffe bekannt.

Als metallische Werkstoffe haben Chrom/Kobalt-Stähle und Titanlegierungen Eingang in die Prothetik gefunden. Beide Werkstoffe sind im wesentlichen biokompatibel, d.h. körperverträglich. Chrom/Kobalt-Stähle finden Verwendung als Werkstoff für Endoprothesen, da sich diese Stähle beständig gegen körpereigene Flüssigkeiten mit einer Wasserstoffionenkonzentration konstant nahe 7 (pH7 = neutral) gezeigt haben. Diese Beständigkeit ist hingegen nicht im Mundbereich mit starken pH-Wert-Schwankungen gegeben. Titanlegierungen (TiAL6N, TiAL6Nb) sind im Gegensatz zu Chrom/Kobalt-Stählen pH-Wert schwankungsresistent. Sie finden somit als Werkstoffe für Endoprothesen und für Prothesen im Mundbereich (Exoprothesen) Anwendung, ihr Nachteil liegt jedoch im Vergleich zu Chrom/Kobalt-Stählen darin, dass diese Titanlegierungen eine nicht vollständig ausreichende Festigkeit aufweisen. Beide metallische Werkstoffe sind jedoch vergleichsweise leicht mechanisch, d.h. spanabhebend bearbeitbar.

Alternativ zu den metallischen haben sich für Endo- und Exoprothesen auch keramische Werkstoffe qualifiziert. An keramische Werkstoffe sind folgende Anforderungen zu stellen. Sie müssen bioinert, d.h. beständig gegen Körperflüssigkeiten sein. Zur Vermeidung der Aufnahme von Körperflüssigkeiten dürfen keramische Prothesen, insbesondere Endoprothesen nicht porös sein. Weiter wird Korrosionsbeständigkeit gefordert, d.h. oberflächlicher Angriff oder Abtrag der Prothesen ist auszuschliessen. Als weitere Anforderungen sind zu stellen eine dem Verwendungszweck der Prothese entsprechende Festigkeit und Biokompatibilität. Keramische Werkstoffe haben vorstehende Anforderungen gesamthaft zu erfüllen, Nichterfüllung einer Anforderung lässt einen keramischen Werkstoff prothetisch ausser Betracht fallen.

Für lasttragende Endo- und Exoprothesen haben sich zwei keramische Werkstoffe qualifiziert, nämlich Aluminiumoxid (AL203) mit einem AL203 Anteil von 99,85%, Rest andere Bestandteile, und Zirkonoxid (ZrO2) in überwiegend tetragonaler Struktur, stabilisiert durch Magnesiumoxid (MaO2) oder durch ein Oxid der seltenen Erden, vorzugsweise Yttriumoxid (Y203) oder Ceroxid (Ce02).

Der Verwendung dieser vorstehend genannten keramischen Materialien sind in der Prothetik deutliche Grenzen gezogen, indem sie zur Herstellung kompliziert dreidimensional ausgebildeter Prothesen, z.B. Zahn-, Finger-, Rückgratteilprothesen etc. wenig geeignet erscheinen. Vorgesinterte AL203 Prothesenhalbzeuge, die nach Fertigbearbeitung dichtgesintert werden müssen, zeichnen sich durch grosse Härte und sehr schwere maschinelle Bearbeitbarkeit aus, was dazu führte, dass AL203 im Dentalbereich - Prothesen, beispielsweise Kronen und Brücken, können Wandstärken bis minimal 0,2 mm haben - keinen Eingang fand. Vergleichbar verhält es sich mit Zr02. In gesinterter Form ist es im Vergleich zu AL203 noch schwerer bearbeitbar. In poröser Form ist es vergleichsweise leicht bearbeitbar, es würde sich also zur maschinellen Formgebung komplizierter dreidimensionaler Strukturen, d.h. Körpern in diesem Zustand eignen, wenn in der Prothetik zur Vermeidung der Porösität nicht allgemein Dichtsinterung gefordert wäre. Im Dentalbereich ist neben der Dichtsinterung zur Beseitigung der Porösität die Dichtsinterung zusätzlich für die Ausbildung von Prothesen filigraner Wandstärken und/oder dünner Stege unabdingbar.

Es ist die maschinelle Nacharheiten und Reinigung erfordernde Dichtsinterung vorgefertigter Protheseteile, die auch ZrO2 in seiner Verwendbarkeit zur Herstellung von Prothesen Grenzen gezogen hat, indem relativ grosse lasttragende Prothesen nicht aber kleine, strukturell komplizierte Prothesen, wie Zahn- oder Fingerprothesen mit diesem keramischen Werkstoff herstellbar sind, es liegt mithin für Zirkonoxid (ZrO2) der vorstehend beschriebenen Art ein begrenzetes Anwendungsspektrum vor. Aufgabe der vorliegenden Erfindung ist es, das Anwendungsspektrum von ZrO2 so zu erweitern, dass mit diesem keramischen Werkstoff die prothetische Produktpalette von lasttragenden grossen bis kompliziert geformten Klein- und Grossimplantaten mit möglichst wenigen ZrO2 Werkstoff-Varianten abgedeckt werden kann und überraschenderweise wurde gefunden, dass diese Aufgabe mit der im Anspruch 1 gekennzeichneten Zusammensetzung gelöst werden kann.

Der erfindungsgemässe Werkstoff ist keramisch, bioinert, korrosionsbeständig, er bestitzt gegenüber den genannten metallischen Materialien eine deutlich höhere Festigkeit und ist nachgewiesenermassen biokompatibel. Diese Vorteile werden ergänzt dadurch, dass zur Herstellung von Prothesen der erfindungsgemässe Werkstoff zunächst zu einem dichtgesinterten Halbzeug verarbeitet werden kann, aus dem anschliessend eine Prothese durch maschinelle Bearbeitung herstellbar ist.

Bei Verwendung des erfindungsgemässen Werkstoffes kann die Dichtsinterung zwar nicht vermieden werden, sie wird aber im Gegensatz zur derzeit herrschenden Praxis im Fertigungsablauf einer Prothese in Richtung eines Halbzeuges verschoben, bei dem die Probleme der Schwindung, Nacharbeit, Säuberung von Verunreinigungen ohne Belang sind.

### Beispiel:

92,1% bis 93,5% ZrO2 werden mit 4,5% bis 5,5% Y2O3 und 1,8% bis 2,2% HfO2, wobei alle Verunreinigungen (Sio2, AL203, T102) höchstens 0,2% (alle %-Angaben sind Gewichts%) gemischt zu einem Halbzeug, z.B. einer Platte oder Rondelle geformt und das Halbzeug wird anschliessend dicht gesintert. Das Halbzeug zeichnet sich durch eine hohe Festigkeit, mittlere Zähigkeit und kleine Korngrösse aus. Durch maschinelle Bearbeitung kann das Halbzeug zu Endo- oder Exoprothese grosser oder kleinster Dimensionierung und komplizierter Struktur und filigraner Wandungsausbildung umgestaltet werden.

## Patentansprüche

1. Yttriumoxid stabilisiertes, in überwiegend tetragonaler Form vorliegendes Zirkonoxid als Mischung der Zusammensetzung ZrO2 92,1% bis 93,5%, Y203 4,5% - 5,5%, HfO2 1,8% bis 2,2%, Verunreinigungen höchstens 0,2% zur Herstellung dichtgesinterten Halbzeuges wie Platten, Rondellen oder dergleichen als Ausgangsmaterial für Prothesen.

## Claims

1. An yttrium-stabilized zirconium oxide in predominantly tetragonal form is of the composition ZrO2 92.1% to 93.5%, Y203 4.5% to 5.5%, HfO2 1.8% to 2.2% impurities at most 0.2% for the production of a densely sintered semi-finished article like plates and rounds or the like as starting material for a prothesis.

## Revendications

1. Oxyde d'yttrium, oxyde de zirconium stabilisé présent en prépondérance sous forme tétragonale, en tant que mélange de composition: ZrO₂ de 92,1% à 93,5%, Y₂O₃ de 4,5% à 5,5%, HfO₂ de 1,8% à 2,2%, impuretés 0,2% au plus, pour la préparation de produits semi-finis frittés de façon dense tels que des disques, des pièces rondes ou similaires en tant que matière première pour des prothèses.
